# EUROPÄISCHE PATENTSCHRIFT

(11) **EP 2 349 149 B1**
(45) Veröffentlichungstag und Bekanntmachung des Hinweises auf die Patenterteilung: **26.04.2017**
(21) Anmeldenummer: 09748049.5
(22) Anmeldetag: 04.11.2009
(51) Int. Cl.: A61F 9/008, A61F 9/01, A61B 3/107, A61F 9/009

(54) **OPHTHALMOLOGISCHES LASERSYSTEM**
OPHTHALMOLOGIC LASER SYSTEM
SYSTÈME OPHTALMOLOGIQUE À LASER

(30) Priorität: 06.11.2008 DE 102008056488
(43) Veröffentlichungstag der Anmeldung: 03.08.2011
(73) Patentinhaber: Carl Zeiss Meditec AG, 07745 Jena (DE)
(72) Erfinder: BISCHOFF, Mark, 07749 Jena (DE); DICK, Manfred, 07926 Gefell (DE); LEDERMANN, Jürgen, 07747 Jena (DE)
(74) Vertreter: Loritz, Rainer
(86) Internationale Anmeldenummer: PCT/EP2009/007892
(87) Internationale Veröffentlichungsnummer: WO 2010/051975

(56) Entgegenhaltungen:
- EP-A1- 1 806 116
- EP-A2- 0 983 757
- WO-A2-2009/146906
- DE-A1- 10 207 535
- DE-A1-102005 030 347
- US-A- 5 092 863

## Beschreibung

Die Erfindung betrifft ein ophthalmologisches Lasersystem, insbesondere zum Zwecke einer chirurgischen Nachbehandlung der Cornea, mit einem Laser, dessen Strahlung als Beleuchtungslicht über einen Beleuchtungsstrahlengang, der eine Scannereinheit und eine Fokussieroptik aufweist, in einem Untersuchungsbereich fokussierbar ist. Die Erfindung betrifft darüber hinaus ein Betriebsverfahren für ein ophthalmologisches Lasersystem.

In der Ophthalmologie ist es bekannt, die Hornhaut (Cornea) des menschlichen Auges, die etwa 500 µm dick ist, bei einer Fehlsichtigkeit durch Abtragen (Ablation) von Gewebe zu formen und damit Myopie, Hyperopie und Astigmatismus zu korrigieren. Dies wird als refraktive Chirurgie bezeichnet. Sie wird heutzutage einerseits mit mechanischen Mitteln wie Mikrokeratomen in Kombination mit Laserstrahlung und andererseits rein optisch mit Laserstrahlung durchgeführt. Bewährt hat sich hierfür die Laserstrahlung des ArF-Excimerlasers, dessen gepulste Strahlung eine Wellenlänge von 193 nm aufweist. Mit Strahlung dieser Wellenlänge werden gute Abtragungsergebnisse bei geringsten, vernachlässigbaren Nebenwirkungen erzielt.

Dabei sind zwei unterschiedliche Verfahren zur Durchführung einer derartigen Operation bekannt. Bei der Photorefraktiven Keratektomie (PRK) wird die obere Epithelschicht, die etwa 50 µm dick ist, von der Bowmanschen Membran mit Hockeymessern irreversibel entfernt und die Laserablation auf der stromalen Oberfläche durchgeführt. Innerhalb eines Heilungsprozesses bildet sich nach der Operation neues Epithel auf der laserbehandelten Oberfläche. Dieses ist jedoch mit Schmerzen für den Patienten verbunden.

Bei der lasergestützten intrastromalen Keratomileusis (LASIK) wird mit Hilfe eines mechanischen Mikrokeratoms ein stromaler Lappen (engl. "flap") mit einer Dicke von etwa 160 µm von der Hornhaut gelöst und um eine nicht abgelöste Stelle in Art eines Scharniers (engl. "hinge") zurückgeklappt. Der Flap wird dabei in der Regel konzentrisch mit der Pupille erzeugt. Die Laserbehandlung wird im intrastromalen Gewebe durchgeführt. Der Flap wird nach der Behandlung zurückgeklappt. Dabei haben die Patienten nach der Operation geringste Schmerzen und eine schnelle Visuserholung. Der Eingriff mit einem Mikrokeratom ist jedoch risikobehaftet und die zur refraktiven Korrektur verfügbare Restdicke der Hornhaut ist geringer als bei der PRK.

Neuerdings wird stark fokussierte Strahlung von Femtosekunden-Lasern verwendet, um in der Cornea Schnitte zu führen (Femto-LASIK). Derartige Geräte werden auch als Lasermikrokeratom bezeichnet. Dabei wird im Fokus eine Photodisruption erzeugt, welche zu einer minimalen Blasenbildung im stromalen Gewebe führt. Setzt man mittels eines Scannersystems Fokuspunkt (engl. "spot") an Fokuspunkt, so kann man beliebige Schnitte (Perforationen) in die Cornea einbringen. Solche Schnitte werden nachfolgend als Laserschnitte bezeichnet. Es ist beispielsweise aus US 2006/0155265 A1 (Intralase Corp.) bekannt, den Flap mittels eines Femtosekunden-Lasersystems zu schneiden. Die Abtragung des für eine refraktive Korrektur erforderlichen stromalen Gewebes erfolgt dann konservativ mittels eines Excimerlasers, so dass auf eine mechanische Bearbeitung vollständig verzichtet werden kann. Jedoch sind zwei Lasersysteme erforderlich.

In WO 2008/064771 A1 (Carl Zeiss Meditec AG) wird ein Femtosekunden-Lasersystem beschrieben, welches ebenfalls den Flap präparieren kann, aber zusätzlich in der Lage ist, den für eine refraktive Korrektur erforderlichen Abtrag stromalen Gewebes durch eine zweifache Schnittführung zur Präparation eines Lentikels zu separieren. Dies kann als Femtosekunden-Lentikel-Extraktion (FLEx) bezeichnet werden. Das Lentikel kann dann nach dem Öffnen des Flaps mit Hilfe einer Pinzette entnommen werden. Dadurch ist nur ein Lasersystem erforderlich, auf einen Excimerlaser kann verzichtet werden.

In einigen Fällen der refraktiven Laserkorrektur von Augen kann es aufgrund von Fehlbehandlungen oder Änderungen des Refraktionszustandes erforderlich werden, eine wiederum lasergestützte Nachbehandlung durchzuführen. Dabei ist der Flap im stromalen Bett oft wieder leicht mechanisch zu lösen, allerdings ist in der Regel am Umfang des Flaps in Abhängigkeit der verstrichenen Zeit seit der vorhergehenden Behandlung Epithel aufgewachsen. Um eine möglichst komplikationsarme Nachbehandlung durchführen zu können, sollten die ursprünglichen Trennstellen sehr genau wieder genutzt werden. Bei einer mechanischen Repräparation eng benachbarter Schnitte besteht jedoch die Gefahr, dass im Vergleich zu einem Altschnitt unabsichtlich zusätzliches oder weniger Gewebe entnommen wird.

Die Repräparation des Flaps ist grundsätzlich problematisch, da biomechanische Veränderungen nach der ersten Laserbehandlung, insbesondere Regressionsprozesse, die Lage der Schnitte bezüglich der als Referenz fungierenden Homhautvorderseite verändert haben können. Folglich sind bei der Nachbehandlung Komplikationen durch eine ungenaue Repräparation nahezu unvermeidbar, wenn die vorgenannten Einflüsse nicht aus anderen Gründen vernachlässigt werden können (beispielsweise wenn die Nachbehandlung binnen kürzester Zeit nach der Erstbehandlung erfolgt).

Diese Problematik tritt nicht nur bei der Repräparation des Flaps, sondern im Fall der Femtosekunden-Lentikel-Extraktion unter Umständen auch bei der eigentlichen refraktiven Nachbehandlung auf, wenn frühere Laserschnitte weitergenutzt werden sollen. Wenn beispielsweise eine derartige Lentikelentnahme minimalinvasiv geschieht, indem das Lentikel durch einen schmalen Randeinschnitt entnommen wird (engl. "Small Incision Lenticle Extraction"; SMILE), ohne einen vollständigen Flap zu öffnen, kann es für eine Nachbehandlung erforderlich werden, im Anschluss an diesen Randeinschnitt eine vollständige Randöffnung für einen Flap zu präparieren und dabei ein Hinge am Rand an einer vorher unbearbeiteten Randzone stehenzulassen.

Aus WO 2006/051364 A1 (20/10 Perfect Vision Optische Geräte GmbH) ist eine weitere Möglichkeit bekannt, um Fehlsichtigkeit laserchirurgisch zu verringern. In diesem Verfahren werden mit einem Femtosekunden-Laser Schnitte in stromalem Gewebe vorgenommen, um ohne Ablation von Gewebe einen zusammenhängenden Hohlraum zu schaffen, insbesondere mit zylindrischer Form. Beim Zusammenbrechen des Hohlraums durch den intraokularen Druck entspannt sich die Cornea und nimmt eine neue Form mit veränderter Krümmung an. Im Grundsatz ähnliche Verfahren sind die radiäre Keratotomie (Fjodorov) und die astigmatische Keratotomie. Durch entsprechende Platzierung von Entspannungsschnitten, was auch laserchirurgisch erfolgen kann, kann eine Fehlsichtigkeit verringert werden. Bei diesen Verfahren ist es jedoch schwierig, eine unvollständige Behandlung, eine Fehlbehandlung oder eine Unter- oder Überkorrektur unmittelbar zu erkennen. Hingegen fällt beim Femto-LASIK-Verfahren beispielsweise ein sich aufgrund unzureichender Schnitte nicht ablösender Flap unmittelbar auf, so dass eine sofortige, komplikationslose Nachbehandlung eingeleitet werden kann. Auch bei den vorgenannten Verfahren können daher eine oder mehrere Nachbehandlungen notwendig sein, beispielweise, um eine Verbesserung der Fehlsichtigkeit iterativ zu erreichen. In der Zwischenzeit können jedoch die oben beschriebenen biomechanischen Veränderungen eintreten.

Der Erfindung liegt die Aufgabe zugrunde, ein ophthalmologisches Lasersystem und ein zugehöriges Betriebsverfahren der eingangs genannten Art so zu verbessern, dass eine Nachbehandlung der Cornea nach einer lasergestützten Erstbehandlung mit hoher Genauigkeit ermöglicht wird.

Die Aufgabe wird gelöst durch ein ophthalmologisches Lasersystem, welches die in Anspruch 1 angegebenen Merkmale aufweist, und durch ein Verfahren, welches die in Anspruch 9 angegebenen Merkmale aufweist.

Vorteilhafte Ausgestaltungen der Erfindung sind in den Unteransprüchen angegeben.

Erfindungsgemäß sind ein Strahlteiler im Beleuchtungsstrahlengang und eine Steuereinheit vorgesehen, wobei Strahlung, die den Strahlteiler aus Richtung des Untersuchungsbereichs erreicht, durch eine konfokale Aperturblende als Detektionslicht auf einen Detektor gelangt, und die Steuereinheit eine im Untersuchungsbereich angeordnete Cornea mittels des Lasers bei einer Beleuchtungs-Laserleistung bestrahlen und Detektionslicht mittels des Detektors aufnehmen kann, wobei sie die Cornea dreidimensional abtastet, indem sie sie an mehreren Punkten bestrahlt und Detektionslicht aufnimmt, und anhand des Detektionslichtes einen vorhandenen Laserschnitt in der Cornea identifiziert und Form und/oder Lage des Laserschnitts ermittelt.

Das erfindungsgemäße ophthalmologische Lasersystem mit integriertem konfokalem Messsystem und das erfindungsgemäße Betriebsverfahren ermöglichen durch die Wiedererkennung der aktuellen Position und/oder -lage von bereits vorhandenen Laserschnitten aus einer vorausgegangenen Femtosekunden-Laserbehandlung oder einer LASIK der Cornea die Nutzung der bereits vorhandenen Schnitte im Rahmen einer Wiederholungs- oder Nachbehandlung mit hoher Genauigkeit. Beispielsweise kann die Nachbehandlung exakt zu den Altschnitten ausgerichtet werden, so dass alte Laserschnitte erneut geschnitten oder neue Schnitte mit hoher Genauigkeit relativ zu vorhandenen Laserschnitten positioniert werden können. Dabei wird die Ortung der Laserschnitte erst durch die konfokale Detektion ermöglicht, denn die Intensität des in der Cornea rückgestreuten Lichts ist aufgrund der inhärenten Eigenschaften der Cornea -für eine hohe Abbildungsqualität muss die Streuung möglichst gering sein- sehr niedrig. Nur eine konfokale Detektion unterdrückt Störlicht stark genug, so dass die an Altschnitten erhöhte Streuung nachweisbar und zur Ortung verwendbar ist. Darüber hinaus erlaubt die Erfindung eine In-vivo-Pachymetrie der Cornea, so dass Schichten der Cornea als Bezugssystem zur Orientierung verwendet werden können. Die Erfindung erlaubt auch eine intraoperative 3D-Darstellung der Cornea auf einem Datensichtgerät zur besseren Orientierung des Operateurs.

In einer vorteilhaften Ausgestaltung ist der Strahlteiler ein Polarisationsstrahlteiler, der das Detektionslicht selektiv so auf den Detektor auskoppelt, dass es eine von dem emittierten Beleuchtungslicht verschiedene Polarisationsrichtung aufweist. Ein großer Teil des vom Untersuchungsbereich auf den Strahlteiler treffenden Lichts stammt aus Reflexionen an den optischen Komponenten des Strahlengangs, beispielsweise den Oberflächen der Fokussieroptik, und weist daher dieselbe Polarisationsrichtung auf wie das Beleuchtungslicht. Indem der Strahlteiler nur Licht einer davon verschiedenen Polarisationsrichtung als Detektionslicht zum Detektor leitet, wird solches Störlicht zurückgehalten. In der Cornea rückgestreutes Licht hingegen weist eine veränderte Polarisationsrichtung auf. Die Detektion des in der Cornea rückgestreuten Lichts ist dadurch mit höherer Genauigkeit möglich.

Ein noch größere Signalstärke kann erzielt werden, indem im Beleuchtungsstrahlengang zwischen der Fokussieroptik und dem Untersuchungsbereich ein optisches Phasenverzögerungssystem so angeordnet ist, dass das hindurchfallende Beleuchtungslicht eine mit dem ausgekoppelten Detektionslicht korrespondierende Polarisationsrichtung erhält. Auf diese Weise hat das Störlicht dieselbe Polarisationsrichtung wie die den Laser verlassende Strahlung, während das im Phasenverzögerungssystem modifizierte Beleuchtungslicht, das die Cornea erreicht, eine definierte andere Polarisationsrichtung erhält. Durch die Selektion des Lichts dieser Polarisationsrichtung als Detektionslicht mittels des Polarisationsstrahlteilers wird nahezu ausschließlich solches Licht detektiert, das in der Cornea rückgestreut wurde. Störlicht, das aus Reflexionen an optischen Komponenten stammt, wird noch effektiver vom Detektor ferngehalten.

Aufgrund der Polarisationseigenschaften der Cornea sind darüber hinaus zur Bildgeneration innerhalb unterschiedlicher Gebiete der Cornea unterschiedliche Polarisationseigenschaften der eingebrachten Diagnosestrahlung vorteilhaft. Diese können durch ein oder mehrere polarisationsoptische Elemente im Beleuchtungsstrahlengang erzeugt werden.

Vorteilhafterweise ist im Detektionsstrahlengang zwischen dem Strahlteiler und dem Detektor ein Polarisationsfilter angeordnet, der hinsichtlich seiner Polarisationsrichtung drehfest oder drehbar ist. Ein drehfester Polarisationsfilter entspricht in der Wirkung dem oben beschriebenen Polarisationsstrahlteiler. Wegen der Polarisationseigenschaften der Cornea ist zur effizienten Diagnose spezieller Gebiete der Cornea eine Selektion der auf den konfokalen Detektor rückgestreuten Polarisationsrichtung durch eine Verdrehung des dem Detektor zugeordneten Polarisationsfilters vorteilhaft. Eine vollständige Detektion eines Gesamtbildes mit hohem Kontrast eines Altschnittes in der Cornea gelingt dann durch eine mehrfache Abtastung (engl. "scanning") bei unterschiedlichen Einstellungen des Polarisationsfilters in ein jeweiliges Einzelbild und entsprechender Überlagerung der Einzelbilder zum Gesamtbild. Auch ein Polarisationsstrahlteiler kann drehbar ausgeführt sein, um Streulicht unterschiedlicher Polarisationsrichtungen selektiv zu detektieren. Die Ortung eines Altschnitts ist jedoch auch mit drehfestem Polarisationsfilter/Polarisationsstrahlteiler oder völlig ohne Polarisationsfilterung möglich, insbesondere mittels eines einzelnen Abtastdurchgangs. Ein einzelner Abtastdurchgang kann in kurzer Zeit durchgeführt werden.

Bevorzugt sind Ausführungsformen, in denen die Strahlung des Lasers neben der Beleuchtungs-Laserleistung auf eine chirurgische Therapie-Laserleistung einstellbar ist. Dadurch ist derselbe Laser sowohl für die Beleuchtung bei der Ermittlung von Form und/oder Lage der Laserschnitte in der Cornea als auch bei der anschließenden Behandlung mit hoher Positioniergenauigkeit verwendbar.

Vorteilhafterweise kann die Steuereinheit im Anschluss an das Ermitteln von Form und/oder Lage der Altschnitte in der Cornea Bestrahlungssteuerdaten für eine lasergestützte chirurgische Nachbehandlung unter Berücksichtigung der ermittelten Form und/oder Lage der Laserschnitte ermitteln und die Cornea bei einer chirurgischen Therapie-Laserleistung gemäß den ermittelten Bestrahlungssteuerdaten bestrahlen. Dadurch ist die Nachbehandlung mit hoher Genauigkeit möglich, da die tatsächliche, aktuelle Lage der Altschnitte deren Wiederverwendung oder Weiterbildung/Fortsetzung ermöglicht. Fehler bei der Wiederverwendung oder Weiterbildung/Fortsetzung von Altschnitten aufgrund einer biomechanischen Veränderung können so minimiert werden.

Zweckmäßigerweise fixiert die Steuereinheit ein die Cornea enthaltendes Auge vor dem Bestrahlen mit Beleuchtungs-Laserleistung mittels einer Fixiervorrichtung und löst die Fixierung des Auges nach dem Ermitteln von Form und/oder Lage der Laserschnitte oder nach einer chirurgischen Bestrahlungsbehandlung.

Zur Verbesserung des Bildkontrastes kann vorteilhaftweise ein mit dem Laser gekoppelter Lock-In-Verstärker für den Detektor vorgesehen werden. Dies ermöglicht die Aufnahme des Detektionslichtes mit hoher Empfindlichkeit, so dass eine eventuelle zugeordnete Behandlung mit hoher Genauigkeit durchgeführt werden kann.

Beim dreidimensionalen Abtasten der Cornea kann die Strahlenbelastung reduziert werden, indem sich zwei aufeinanderfolgende Abtastpunkte in allen drei Raumkoordinaten unterscheiden. Durch diese Art der Abtastung kann ein repräsentatives Modell der Cornea hinsichtlich Form und/oder Lage von Altschnitten in kurzer Zeit gewonnen werden. Technisch besonders vorteilhaft ist eine Ansteuerung der Scanner in Form von Sinus-Funktionen. Bei einer Ansteuerung der x-y-Scanner in der Form, dass der eine Scanner mit genau der doppelten Frequenz des anderen angesteuert wird, ergibt sich beispielweise eine Lissajous-Figur, die einer Acht ähnelt.

Vorzugsweise wird eine Pulsfrequenz des Laserlichts in Abhängigkeit der Bewegungsgeschwindigkeit eines Fokuspunkts des Laserstrahls relativ zur Cornea eingestellt. Dadurch kann bei der Identifikation und Ortung der Altschnitte und insbesondere bei der Nachbehandlung die Strahlenbelastung der Cornea und des Auges insgesamt verringert werden.

Vorteilhafterweise kann der Schritt des Bestrahlens und Abtastens der Cornea entlang einer Abtastkurve mehrfach durchgeführt werden, wobei die Abtastkurve in den Abtastdurchgängen jeweils in unterschiedlich versetzter und/oder unterschiedlich rotierter Lage verwendet wird. Da sich bei einer Tiefen- und Seiten-Abtastung durch das konfokale Detektionssystem nur ein Schnittbild der Cornea ergibt, gelingt eine möglichst vollständige dreidimensionale Analyse eines Altschnittes beispielsweise durch eine Wiederholung mit einer in Schritten von 1°,5°, 10°, ... 90° zentralsymmetrisch zum ersten Abtastdurchgang rotierten Abtastkurve.

Zweckmäßigerweise wird von aufgenommenem Detektionslicht einen Dunkelfeldwert abgezogen. Es kann sich dabei um einen gemeinsamen Dunkelfeldwert für alle Abtastpunkte oder um mehrere, punktspezifische Dunkelfeldwerte handeln. Diese Ausgestaltung ermöglicht eine höhere Genauigkeit der Aufnahme des in der Cornea rückgestreuten Lichts.

Die Erfindung umfasst neben dem ophthalmologischen Lasersystem und einem Betriebsverfahren für ein solches auch ein Computerprogramm sowie eine Steuereinheit, die zur Durchführung eines erfindungsgemäßen Betriebsverfahrens eingerichtet sind.

Stammen Altschnitte aus einer minimalinvasiven Behandlung wie SMILE, können diese mittels der Erfindung, wenn gewünscht, in eine der klassischen LASIK ähnliche Schnittgeometrie umgewandelt werden. Dabei können bei der primären Behandlung bereits angelegte Schnitte erweitert werden. So kann beispielsweise ein primärer Schnitt, welcher im Wesentlichen in konstantem Abstand zur Hornhautvorderseite angelegt wurde, radial erweitert werden, um so günstigere Voraussetzungen für eine Nachbehandlung zu schaffen. Die Erweiterung baut zweckmäßigerweise auf eine vorherige Identifikation und Lokalisierung des Altschnittes/der Altschnitte auf.

Nachfolgend wird die Erfindung anhand von Ausführungsbeispielen näher erläutert.

In den Zeichnungen zeigen:
Fig. 1 ein ophthalmologisches Lasersystem zur Analyse der Cornea,
Fig. 2 ein ophthalmologisches Lasersystem zur Analyse und Bearbeitung der Cornea,
Fig. 3 ein Ablaufdiagramm eines Betriebsverfahrens und
Fig. 4 eine Raumkurve für die Abtastung der Cornea.

In allen Zeichnungen tragen übereinstimmende Teile gleiche Bezugszeichen.

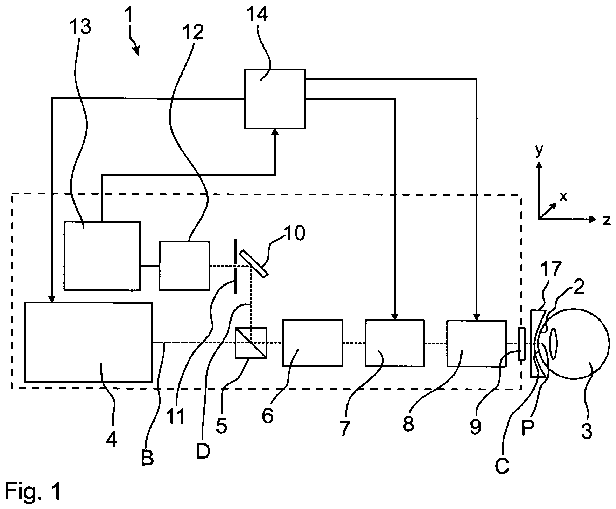

Fig. 1 zeigt ein beispielhaftes ophthalmologisches Lasersystem 1 zur Identifikation und Ortung eines vorhandenen Laserschnitts C in einer Cornea 2 eines Auges 3. Das Lasersystem 1 umfasst einen Laser 4, einen Polarisationsstrahlteiler 5, eine Scanoptik 6, eine Scannereinheit 7, eine Fokussieroptik 8 und ein optisches Phasenverzögerungssystem 9, die zusammen einen Beleuchtungsstrahlengang B bilden, sowie einen Umlenkspiegel 10, eine konfokale Aperturblende 11 und einen Detektor 12, die einen ausgekoppelten Detektionsstrahlengang D bilden. Daneben umfasst das Lasersystem 1 einen Verstärker 13 und eine Steuereinheit 14. Zwischen dem Lasersystem 1 und dem Auge 3 ist ein Kontaktglas 17 mit einer Fixiervorrichtung für das Auge 3 angeordnet, hinter dem der Untersuchungsbereich liegt. Andere Ausführungsformen zur Realisierung der erfindungsgemäßen Lösung sind möglich (nicht abgebildet).

Der Laser 4 ist beispielsweise als gepulster TiSa-Infrarot-Laser mit einer Pulslänge zwischen 100 fs und 1000 fs ausgebildet. Er emittiert Laserstrahlung bei einer augensicheren Beleuchtungs-Laserleistung im Bereich von 100 mW. Die Scannereinheit 7 umfasst beispielsweise eine Anzahl von galvanometrischen Spiegeln zur Ablenkung der Laserstrahlung in x- und y-Richtung über die Cornea 2. Die Fokussierung der Laserstrahlung in z-Richtung entlang der optischen Achse gelingt beispielsweise durch eine bewegliche Linse oder Linsengruppe innerhalb der Scanoptik 6 oder der Fokussieroptik 8 oder alternativ durch eine bewegliche Tubuslinse (nicht abgebildet). Das optische Phasenverzögerungssystem 9 ist beispielsweise als λ/4-Platte ausgebildet, die einen Abschluss des Lasersystems 1 bildet. Der Detektor 12 ist beispielsweise als Photovervielfacher (engl. "photomultiplier"; PMT) oder als Lawinenphotodiode (engl. "avalanche photo diole"; APD) ausgebildet, da die aufzunehmenden Lichtintensitäten niedrig sind. Der Verstärker 13 ist als Lock-In-Verstärker ausgebildet und sowohl mit dem Detektor 12 als auch mit dem Laser 4 verbunden.

Die gepulste IR-Laserstrahlung tritt aus dem Laser 4 aus und durchläuft zunächst unverändert den Polarisationsstrahlteiler 5. Sie wird anschließend über die Scanoptik 6, die Scannereinheit 7 und die Fokussieroptik 8 als Beleuchtungslicht auf einen Abtastpunkt P in der Cornea 2 fokussiert. Dieser Abtastpunkt P kann mittels der Scannereinheit 7 und einer beweglichen Linse oder Linsengruppe innerhalb der Scanoptik 6 oder der Fokussieroptik 8 in x-, y- und z-Richtung in der Cornea 2 verschoben werden. Das optische Phasenverzögerungssystem 9 bewirkt dabei eine definierte Veränderung der Polarisationsrichtung des hindurchfallenden Beleuchtungslichts.

An den Grenzflächen und im Inneren der Cornea 2 kommt es zur Streuung/Reflexion der IR-Strahlung, wobei die Strahlung teilweise depolarisiert wird. Rückgestreutes/reflektiertes Licht fällt auch in den Beleuchtungsstrahlengang B und läuft dort den Weg zurück bis zum Polarisationsstrahlteiler 5. Die Strahlungsanteile mit unverändertem Polarisationszustand fallen durch den Polarisationsstrahlteiler 5 hindurch auf den Laser 4. Dies betrifft insbesondere Reflexe, die von der Scanoptik 6 oder der Fokussieroptik 8 stammen. Solche Strahlungsanteile, die nach Durchlaufen des Phasenverzögerungssystems 9 beziehungsweise durch Depolarisation im Auge 3 in der Cornea 2 einen veränderten Polarisationszustand aufweisen, werden von dem Polarisationsstrahlteiler 5 als Detektionslicht in den Detektionsstrahlengang D zum Detektor 12 abgelenkt. Das Detektionslicht fällt über einen Umlenkspiegel 10 durch die konfokale Lochblende 11 auf den Detektor 12. Der Umlenkspiegel 10 kann in einer alternativen Ausführungsform (nicht abgebildet) entfallen oder durch andere Strahlführungseinheiten ersetzt werden. Die Konfokalblende 11 wirkt als Diskriminator in z-Richtung, so dass ortsaufgelöst nur rückgestreutes Licht aus einem geringen Fokusvolumen detektiert wird. Die Steuereinheit 14 kann durch Ablenken des Beleuchtungslichts in x- und y-Richtung mittels der Scannereinheit 7 und Veränderung der Fokussierung in z-Richtung mittels der Fokussieroptik 8 beliebige Abtastpunkte P innerhalb der Cornea 2 mit Beleuchtungslicht bestrahlen und über die Intensität des zugehörigen Detektionslichtes die Stärke der Rückstreuung an diese Punkten P ermitteln.

Um in kurzer Zeit eine Information über Vorhandensein sowie Form und Lage von Altschnitten in der Cornea 2 mit hoher Genauigkeit zu ermitteln, wird eine geeignete räumliche Verteilung von Punkten P abgetastet. Aus den dabei gewonnen Werten für die Stärke der Rückstreuung können Form und Lage der Altschnitte rekonstruiert werden, da die Rückstreuung an den Grenzflächen der Altschnitte und in deren Inneren gegenüber der unbehandelten Cornea verstärkt ist. Dadurch kann eine Nachbehandlung unter Berücksichtigung der Altschnitte patientenangepasst durchgeführt werden.

In der abgebildeten Ausführungsform bewirkt das optische Phasenverzögerungssystem 9 zwischen dem Auge 3 und der Fokussieroptik 8 eine definierte Drehung der Polarisationsrichtung der hindurchfallenden Beleuchtungslichts, während zuvor an den optischen Komponenten reflektiertes Störlicht die ursprüngliche Polarisationsrichtung beibehält. Dadurch wird die relative Intensität des Detektionslichtes erhöht, da der Polarisationsstrahlteiler 5 nur Licht mit abweichender Polarisationsrichtung als Detektionslicht separiert. In alternativen Ausführungsformen (nicht abgebildet) kann auf das optische Phasenverzögerungssystem 9 verzichtet werden. Alternativ oder zusätzlich können weitere Polarisatoren (nicht abgebildet) in dem Beleuchtungs- und/oder Detektionsstrahlengang angeordnet werden, um die Signalqualität zu verbessern.

Das Phasenverzögerungssystem kann in einer Ausführungsform als Depolarisator realisiert werden, so dass über den Strahlquerschnitt die Größe der Phasenverzögerung variiert.

Da die am Detektor 12 registrierten Signale eine sehr geringe Intensität aufweisen, ist der elektronische Verstärker für ein optimiertes Signal-Rausch-Verhältnis angepasst. Eine besonders vorteilhafte Ausführungsform ist der Lock-In-Verstärker, der zeitlich mit der Pulsgenerierung beziehungsweise mit der Folgefrequenz des Lasers 2 synchronisiert ist. Andere Ausführungsformen verwenden beispielsweise sogenannte "Boxcar"-Techniken oder Abtasttechniken (engl. "sampling") mit Aufsummierung oder Mittelung zur Rauschunterdrückung. Vorteilhafterweise weist das gesamte Verstärkersystem des Detektorsignals eine nichtlineare Kennlinie auf. Es kann auch ein Spitzen-Detektor (engl, "peak detector") und/oder eine Abtast-Halte-Schaltung (engl. "sample and hold") verwendet werden, um eine Signalverbesserung zu erreichen.

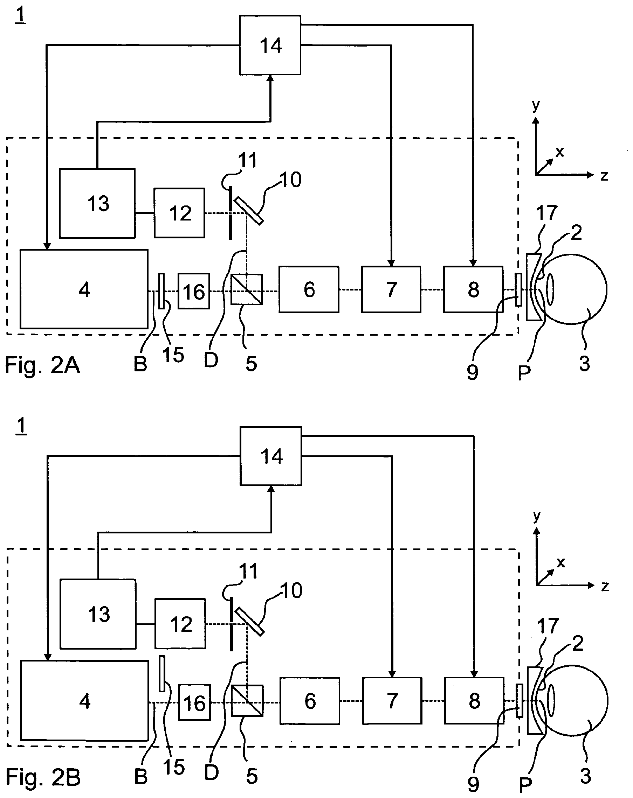

In Fig. 2 ist ein ophthalmologisches Lasersystem 1 zur kombinierten Identifikation von Altschnitten einer Erstbehandlung und Durchführung einer lasergestützten Nachbehandlung dargestellt. Es entspricht weitgehend dem Lasersystem 1 gemäß Fig. 1, ist aber zusätzlich mit einem in den Beleuchtungsstrahlengang B einklappbaren Abschwächer 15 und einem Modulator 16, beispielsweise einem akustooptischen Modulator, ausgerüstet. Der Abschwächer 15 dient zur Umschaltung zwischen einer Beleuchtungs-Laserleistung und einer Therapie-Laserleistung. Die Beleuchtungs-Laserleistung wird mit in den Beleuchtungsstrahlengang B eingeklapptem Abschwächer 15 erzielt, die Therapie-Laserleistung ohne Abschwächer 15. Die optischen Komponenten, insbesondere die Optiken 6 und 8, sind auf das Ziel einer bestmöglichen Fokusminiaturisierung optimiert, korrigiert und untereinander abgestimmt. Beispielsweise sind ihre optischen Aberrationen hochgradig minimiert, so dass für eine Photodisruption nur ein geringer Energieeintrag erforderlich ist.

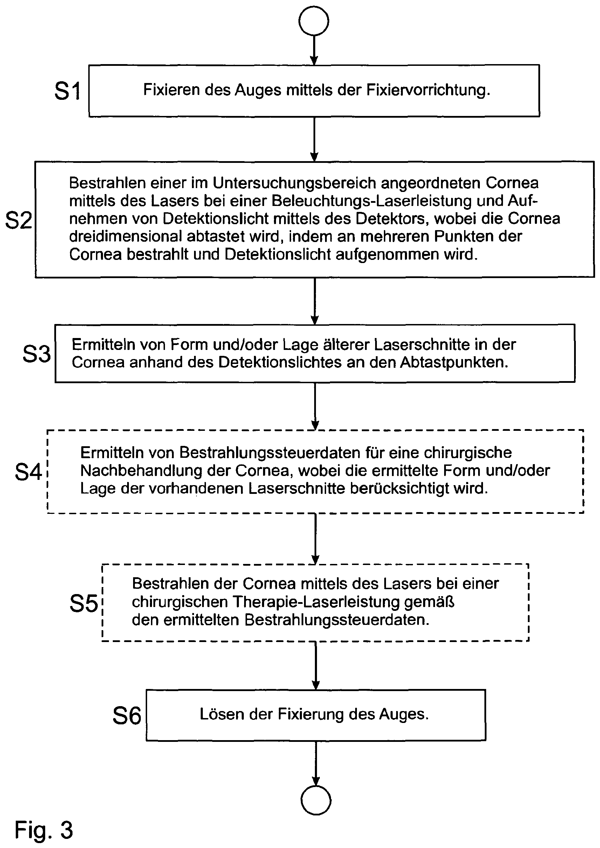

Die Steuereinheit 14 führt das in Fig. 3 dargestellte Betriebsverfahren durch, wobei für eine reine Ortung von Altschnitten in der Cornea 2 nur die durchgezogen umrandeten Schritte S1, S2, S3 und S6 durchgeführt werden. Für eine Nachbehandlung werden alle Schritte durchgeführt. Der Laser 4 wird dabei sowohl für die Beleuchtung während der Detektionsphase als auch für die Bearbeitung der Cornea 2 während der sich unmittelbar anschließenden Behandlungsphase verwendet.

Zunächst wird das Patientenauge 3 fixiert, beispielsweise mittels Unterdruck an eine Kontaktglasvorrichtung angesaugt (Schritt S1). Zusätzlich kann der Kopf des Patienten fixiert sein. Der Blick des Patienten kann durch ein geeignetes Ziel möglichst konstant gehalten werden. Dabei ist eine einstellbare Kompensation des Winkels zwischen Geometrie- und Sehachse des Auges 3 möglich.

Entlang einer einstellbaren kontinuierlichen, dreidimensionalen Abtastkurve oder Abtaststruktur wird das Beleuchtungslicht bei Beleuchtungs-Laserleistung mit einer variablen Pulsfrequenz über die Cornea 2 geführt und Detektionslicht aufgenommen (Schritt S2). Die Pulsfrequenz wird dabei in Abhängigkeit der Geschwindigkeit der Abtastbewegung so eingestellt, dass bei langsamer Abtastbewegung eine niedrigere Pulsfrequenz resultiert als bei schneller Abtastbewegung. Einzelnen Punkten der Abtastkurve wird das rückgestreute Detektionslicht abschnitts- oder punktweise zugeordnet. Durch die Stetigkeit der Abtastkurve unterscheiden sich aufeinanderfolgende Abtastpunkte in allen Raumkoordinaten. Von den detektierten Signalwerten werden vorteilhafterweise jeweilige Dunkelfeldwerte abgezogen werden, die in einem separaten Kalibrierdurchgang ermittelt werden.

Aus den den Abtastpunkten zugeordneten Intensitäten werden Altschnitte identifiziert und deren Form und Lage rekonstruiert (Schritt S3). Zu diesem Zweck werden beispielsweise Abtastpunkte, deren Intensität einen vorgegebenen oder vom Operateur vorgebbaren Intensitätsschwellwert überschreitet, als Stützstellen des Altschnitts bestimmt. In einer Ausgleichsrechnung ein angenommenes oder aus der früheren Behandlung bekanntes Modell des Altschnittes an die dreidimensionalen Koordinaten der ermittelten Stützstellen des Altschnittes angepasst, um sämtliche Koordinaten des Altschnittes als Basis für die chirurgische Nachbehandlung des Altschnittes verfügbar zu machen. Diese Informationen werden verwendet, um die beispielsweise zuvor vom Operateur vorgegebenen, zu führenden Schnitte an den tatsächlichen, individuellen Zustand der Cornea 2 anzupassen, bevor die Bestrahlungssteuerdaten bestimmt werden (Schritt S4). Die Bestrahlungssteuerdaten umfassen beispielsweise Ansteuersignale für die Achsen der Scannereinheit 7 beziehungsweise für die interne z-Fokussierung und für die Laserstrahlquelle und den Leistungsmodulator 16. Falls die in der Erstbehandlung erzeugten Schnittgeometrien bekannt sind, bedarf es mitunter nur einer reduzierten Information über die bereits implementierten Schnitte, um die vollständige Lage der Schnitte zu rekonstruieren. Ist beispielsweise lediglich die Zentrierung eines Flap-Schnittes zweifelhaft, so reicht schon die Bestimmung von zwei Randschnittpunkten innerhalb des durch das Kontaktglas bereitgestellten Bezugssystems aus.

Unmittelbar anschließend wird die chirurgische Nachbehandlung bei Therapie-Laserleistung anhand der Bestrahlungssteuerdaten durchgeführt (Schritt S5). Vorteilhafterweise werden Pulsenergien von 10 nJ bis 3 µJ, insbesondere 50 nJ bis 1 µJ, verwendet. Dabei werden beispielsweise durch die Laserstrahlung bei einer Pulsfrequenz von 100 kHz bis 10 MHz und einer Pulslänge von weniger als 1 ps, insbesondere von 100 fs bis 800 fs, eine oder mehrere Folgen von Photodisruptionen erzeugt. Schließlich wird die Fixierung des Auges 3 gelöst (Schritt S6).

Durch den identischen Strahlengang für Analyse und Behandlung ist das System 1 selbstkalibrierend. Da die Bestrahlungssteuerdaten anhand der mit dem identischen Strahlengang ermittelten Informationen über Form und/oder Lage der Altschnitte in der Cornea bestimmt werden, ist die Behandlung stets mit hoher Genauigkeit möglich.

Durch den Einsatz angepasster Abtastkurven (Scanmuster), beispielsweise in Form von Lissajous-Figuren, ist der kombinierte Vorgang zudem in kurzer Zeit möglich, beispielsweise innerhalb von maximal 30 Sekunden, was einerseits Bewegungsungenauigkeiten reduziert und andererseits zu einer höheren Akzeptanz beim Patienten führt.

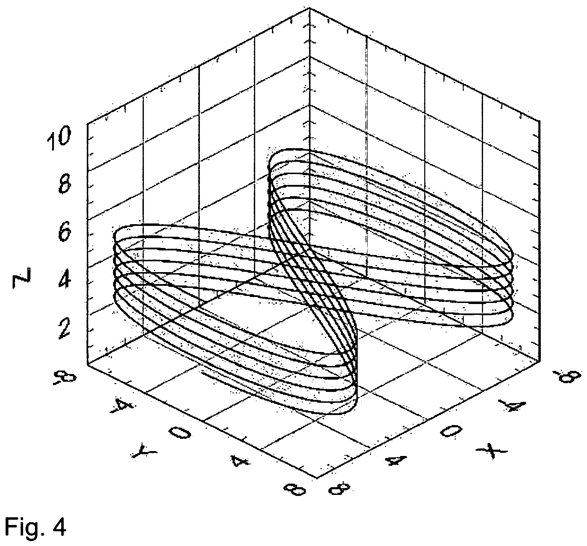

In Fig. 4 ist eine beispielhafte Abtastkurve in Form räumlich versetzter Achten gezeigt, die als Lissajous-Figur mittels der Scannereinheit 6 realisiert werden kann. Sie hat den Vorteil, dass in kurzer Zeit die Daten von vorhandenen Laserschnitten mit hoher Genauigkeit ermittelt werden können.

Andere beispielhafte Formen der Abtastung beziehungsweise Abrasterung können sein (nicht abgebildet): zwei gekreuzte Rechtecke im Raum; zwei Zylinderoberflächen; ein zylindrischer Körper mit Querschnitt in Form einer acht oder vier; mehrere Abtastungen längs eindimensionaler Linien. Möglich ist auch das Abrastern des Volumens eines Zylinders oder eines Würfels. Die Volumina beziehungsweise Oberflächen können kontinuierlich oder auch lediglich teilweise, also mit Zwischenräumen zwischen den einzelnen Abtastpunkten, abgerastert werden. So können zwischen einzelnen Zeilen größere Abstände auftreten.

Nachfolgend werden zwei beispielhafte Anwendungsfälle der Erfindung beschrieben.

### Beispiel 1:

Nachdem beispielsweise bei einem Patienten im Zeitraum von vor einigen Tagen bis mehreren Jahren eine Korrektur der Brechkraft der Hornhaut mit der LASIK-Methode erfolgte, wobei das Flap mit einem Femtosekunden-Lasersystem erzeugt wurde und die eigentliche refraktive Korrektur mit einem Excimerlaser durchgeführt wurde (Femto-LASIK), soll beispielsweise eine laserbasierte Nachkorrektur mit dem Femtosekunden-Lentikel-Extraktions-Verfahren erfolgen. Zu diesem Zweck wird das Patientenauge 3 an das Femtosekunden-Lasertherapiegerät 1 angekoppelt. Dies erfolgt vorzugsweise zentriert zur optischen Achse des Therapiesystems 1. Anschließend wird mit dem konfokalen Detektor 11/12 das oben beschriebene Verfahren derart durchgeführt, dass die Form und Lage der früheren Flap-Schnittfläche bezüglich des mit dem zu behandelnden Auge fest gekoppelten Kontaktglases 17 bestimmt sind. Dazu werden zwei vorzugsweise orthogonale Schnittbilder der zu behandelnden Cornea 2 erzeugt, die sich auf der optischen Achse des Laserstrahls schneiden. Mit oder ohne Zuhilfenahme anderer Information über die ursprüngliche Lage des ursprünglichen Flaps wird im Rahmen der Nachbehandlung die Form und Lage der früheren Flap-Schnittfläche mit einem in gleicher Tiefe positionierten sekundären Flap-Schnitt nachgearbeitet.

Es ist dabei vorteilhaft, aber nicht zwingend, dass die laterale Ausdehnung der Flap-Schnittfläche die ursprüngliche Ausdehnung erreicht oder überschreitet. Ein Teil des früheren Randschnittes sollte mit einem sekundären Randschnitt nachgearbeitet werden. Mitunter ist es aber besser, den sekundären Öffnungsschnitt nicht mit dem ursprünglichen Randschnitt in Deckung zu bringen, etwa bei einem kleinen Behandlungsdurchmesser, wie er beim Femtosekunden-Lentikel-ExtraktionsVerfahren angewendet werden kann. In jedem Fall ist es für den Chirurgen leicht möglich, die ursprünglichen Schnittflächen erneut manuell zu durchtrennen.

Sekundärer Randschnitt und sekundäre Flap-Schnittfläche müssen nicht zueinander zentriert liegen.

Zusätzlich zur sekundären Flap-Schnittfläche wird auch die Lentikel-Schnittfläche erzeugt, die zusammen mit der sekundären Flap-Schnittfläche ein Lentikel einschließt, dessen Entnahme die im Rahmen der Nachbehandlung gewünschte Änderung der Brechkraft der Cornea bewirkt. Mit der abschließenden manuellen Entnahme des Lentikels ändert sich die Brechkraft der Cornea in der beabsichtigten Weise.

### Beispiel 2:

Zunächst wird die Lage der Altschnitte bezüglich des Kontaktglases 17 bei der Nachbehandlung ermittelt (siehe Beispiel 1). Weil aber die Nachkorrektur beispielsweise nur eine geringe Änderung der Brechkraft der Cornea 2 zum Ziel hat, etwa bis zu 2 dpt, werden statt einer Femtosekunden-Lentikel-Extraktion lediglich Entspannungsschnitte ohne Ablation geführt. Eine manuelle Entnahme von Gewebe ist nicht vorgesehen. Erfindungsgemäß wird die Information über die Lage der ursprünglichen Alt-Schnittflächen dazu verwendet, um neue Schnittflächen zu berechnen, die eine Veränderung der Brechkraft der Cornea 2 durch die Veränderung der Form der Vorderseite der Cornea 2 bewirken. Die Form und Lage dieser Schnittflächen kann beispielsweise über eine Finite-Elemente-Rechnung (FEM) oder aus der Erfahrung festgelegt werden.

Nicht nur bei der Begrenzung der Änderung der Amplitude der Brechkraft (geringer Korrekturbetrag), auch bei Einschränkung der optischen Zone, etwa bei der Erzeugung eines Bifokalsystems zur Pseudo-Presbyopie-Korrektur, ist die erfindungsgemäße Nachbehandlungsmethode geeignet.

### Bezugszeichenliste

- 1: Ophthalmologisches Lasersystem
- 2: Cornea
- 3: Auge
- 4: Laser
- 5: Polarisationsstrahlteiler
- 6: Scanoptik
- 7: Scannereinheit
- 8: Fokussieroptik
- 9: Optisches Phasenverzögerungssystem
- 10: Umlenkspiegel
- 11: Konfokale Aperturblende
- 12: Detektor
- 13: Verstärker
- 14: Steuereinheit
- 15: Abschwächer
- 16: Modulator
- 17: Kontaktglas

- B: Beleuchtungsstrahlengang
- D: Detektionsstrahlengang
- P: Abtastpunkt
- C: Laserschnitt

## Patentansprüche

1. Ophthalmologisches Lasersystem (1), insbesondere zum Zwecke einer chirurgischen Nachbehandlung der Cornea, mit einem Laser (4), dessen Strahlung als Beleuchtungslicht über einen Beleuchtungsstrahlengang (B), der eine Scannereinheit (7) und eine Fokussieroptik (8) aufweist, in einem Untersuchungsbereich fokussierbar ist, aufweisend einen Strahlteiler (5) im Beleuchtungsstrahlengang (B), wobei Strahlung, die den Strahlteiler (5) aus Richtung des Untersuchungsbereichs erreicht, durch eine konfokale Aperturblende (11) als Detektionslicht auf einen Detektor (12) gelangt, und eine Steuereinheit, die eingerichtet ist zum:
- Bestrahlen einer im Untersuchungsbereich angeordneten Cornea (2) mittels des Lasers (4) bei einer Beleuchtungs-Laserleistung und Aufnehmen von Detektionslicht mittels des Detektors (12), wobei die Steuereinheit (14) die Cornea (2) dreidimensional abtastet, indem sie sie an mehreren Punkten bestrahlt und Detektionslicht aufnimmt,
**gekennzeichnet dadurch, dass** die Steuereinheit eingerichtet ist zum:
- Identifizieren eines Laserschnitts (C) in der Cornea (2) und Ermitteln von Form und/oder Lage des Laserschnitts (C) anhand des Detektionslichtes.

2. Ophthalmologisches Lasersystem (1) nach Anspruch 1, **dadurch gekennzeichnet, dass** der Strahlteiler (5) ein Polarisationsstrahlteiler ist, der das Detektionslicht selektiv so auf den Detektor (12) auskoppelt, dass es eine von dem emittierten Beleuchtungslicht verschiedene Polarisationsrichtung aufweist

3. Ophthalmologisches Lasersystem (1) nach Anspruch 1 oder 2, **dadurch gekennzeichnet, dass** im Beleuchtungsstrahlengang (B) zwischen der Fokussieroptik (8) und dem Untersuchungsbereich ein optisches Phasenverzögerungssystem (9) so angeordnet ist, dass das hindurchfallende Beleuchtungslicht eine mit dem ausgekoppelten Detektionslicht korrespondierende Polarisationsrichtung erhält.

4. Ophthalmologisches Lasersystem (1) nach Anspruch 1, **dadurch gekennzeichnet, dass** im Detektionsstrahlengang (D) zwischen dem Strahlteiler (5) und dem Detektor (12) ein polarisationsoptisches Element, das hinsichtlich einer Polarisationsrichtung drehfest oder drehbar ist, angeordnet ist.

5. Ophthalmologisches Lasersystem (1) nach einem der Ansprüche 1 bis 4, **dadurch gekennzeichnet, dass** die Strahlung des Lasers (4) neben der Beleuchtungs-Laserleistung auf eine chirurgische Therapie-Laserleistung einstellbar ist.

6. Ophthalmologisches Lasersystem (1) nach Anspruch 5, **dadurch gekennzeichnet, dass** mittels der Steuereinheit (14) nach dem Ermitteln von Form und/oder Struktur und/oder Lage der Cornea (2) folgende Schritte ausführbar sind:
- Ermitteln von Bestrahlungssteuerdaten für eine refraktiv-chirurgische Nachbehandlung, wobei die Steuereinheit (14) die ermittelte Form und/oder Lage der Laserschnitte (C) berücksichtigt, und
- Bestrahlen der Cornea (2) bei einer chirurgischen Therapie-Laserleistung gemäß den ermittelten Bestrahlungssteuerdaten.

7. Ophthalmologisches Lasersystem (1) nach einem der vorhergehenden Ansprüche, **dadurch gekennzeichnet, dass** die Steuereinheit (14) ein die Cornea (2) enthaltendes Auge (3) vor dem Bestrahlen mit Beleuchtungs-Laserleistung mittels einer Fixiervorrichtung (17) fixiert und nach dem Ermitteln von Form und/oder Lage der Laserschnitte (C) oder nach einem chirurgischen Therapieren die Fixierung des Auges (3) löst.

8. Ophthalmologisches Lasersystem (1) nach einem der vorhergehenden Ansprüche, **gekennzeichnet durch** einen mit dem Laser (4) gekoppelten Lock-In-Verstärker (13) für den Detektor (12).

9. Computerprogramm oder Steuereinheit (14) für ein ophthalmologisches Lasersystem (1), dessen Laser (4) zwischen einer Beleuchtungs-Laserleistung und einer Therapie-Laserleistung umschaltbar ist und dessen Laserlicht dreidimensional variabel in einer Cornea (2) fokussierbar ist, nach einem der vorhergehenden Ansprüche, eingerichtet zur Durchführung folgender Schritte:
- Fixieren eines die Cornea (2) enthaltenden Auges (3) mittels einer Fixiervorrichtung (17),
- Bestrahlen einer im Untersuchungsbereich angeordneten Cornea (2) mittels des Lasers (4) bei einer Beleuchtungs-Laserleistung und Aufnehmen von Detektionslicht mittels des Detektors (12), wobei die Cornea (2) dreidimensional abgetastet wird, indem sie an mehreren Punkten bestrahlt und Detektionslicht aufgenommen wird,
- Identifizieren eines Laserschnitts (C) in der Cornea (2) und Ermitteln von Form und/oder Lage des Laserschnitts (C) anhand des Detektionslichtes an den Abtastpunkten (P), wobei Abtastpunkte als Stützstellen des Laserschnitts (C) bestimmt werden,
- Ermitteln von Bestrahlungssteuerdaten für eine refraktiv-chirurgische Nachbehandlung, wobei die ermittelte Form und/oder Lage der Laserschnitte (C) berücksichtigt wird, indem in einer Ausgleichsrechnung ein Modell des Laserschnittes (C) an die dreidimensionalen Koordinaten der ermittelten Stützstellen angepasst wird,
- Bestrahlen der Cornea (2) mittels des Lasers (4) bei einer chirurgischen Therapie-Laserleistung gemäß den ermittelten Bestrahlungssteuerdaten und
- Lösen der Fixierung des Auges (3).

10. Computerprogramm oder Steuereinheit (14) nach Anspruch 9, wobei zwei aufeinanderfolgende Abtastpunkte (P) sich in allen drei Raumkoordinaten unterscheiden.

11. Computerprogramm oder Steuereinheit (14) nach Anspruch 9 oder 10, eingerichtet zum Einstellen einer Pulsfrequenz des Laserlichts in Abhängigkeit der Bewegungsgeschwindigkeit eines Fokuspunkts des Laserstrahls relativ zur Cornea (2).

12. Computerprogramm oder Steuereinheit (14) nach einem der Ansprüche 9 bis 11, wobei eingerichtet zum mehrfachen Durchführen des Schritts des Bestrahlens und Abtastens der Cornea entlang einer Abtastkurve, wobei die Abtastkurve in den Abtastdurchgängen jeweils in unterschiedlich versetzter und/oder unterschiedlich rotierter Lage verwendet wird.

13. Computerprogramm oder Steuereinheit (14) nach einem der Ansprüche 9 bis 12, eingerichtet zum Abziehen eines Dunkelfeldwerts von aufgenommenem Detektionslicht.

## Claims

1. Ophthalmologic laser system (1), in particular for the purpose of surgical follow-up treatment of the cornea, with a laser (4) whose radiation is focusable as illumination light in an examination area via an illumination beam path (B) that has a scanner unit (7) and focusing optics (8), having a beam splitter (5) in the illumination beam path (B), wherein radiation that reaches the beam splitter (5) from the direction of the examination area passes as detection light through a confocal aperture stop (11) to a detector (12), and
a control unit which is configured for:
- irradiation of a cornea (2), arranged in the examination area, by means of the laser (4) with an illumination laser power and mapping of detection light by means of the detector (12), wherein the control unit (14) scans the cornea (2) three-dimensionally by irradiating said cornea at several points and mapping detection light,
**characterized in that** the control unit is configured for:
- identification of a laser incision (C) in the cornea (2) and determination of the form and/or position of the laser incision (C) on the basis of the detection light.

2. Ophthalmologic laser system (1) according to Claim 1, **characterized in that** the beam splitter (5) is a polarization beam splitter, which selectively decouples the detection light to the detector (12) such that the detection light has a polarization direction different from the emitted illumination light.

3. Ophthalmologic laser system (1) according to Claim 1 or 2, **characterized in that** an optical phase retardation system (9) is arranged in the illumination beam path (B) between the focusing optics (8) and the examination area such that the illumination light passing through acquires a polarization direction that corresponds to the decoupled detection light.

4. Ophthalmologic laser system (1) according to Claim 1, **characterized in that** a polarizing optical element, which is fixed in rotation or rotatable with respect to a polarization direction, is arranged in the detection beam path (D) between the beam splitter (5) and the detector (12).

5. Ophthalmologic laser system (1) according to one of Claims 1 to 4, **characterized in that** the laser (4) is adjustable to emit, besides the illumination laser power, a surgical therapy laser power.

6. Ophthalmologic laser system (1) according to Claim 5, **characterized in that**, after the determination of the form and/or structure and/or position of the cornea (2), the following steps can be carried out by means of the control unit (14):
- determination of irradiation control data for refractive surgical follow-up treatment, wherein the control unit (14) takes into account the determined form and/or position of the laser incisions (C), and
- irradiation of the cornea (2) with a surgical therapy laser power in accordance with the determined irradiation control data.

7. Ophthalmologic laser system (1) according to one of the preceding claims, **characterized in that** the control unit (14) uses an immobilizing device (17) to immobilize an eye (3), containing the cornea (2), before the irradiation with illumination laser power, and, after the determination of the form and/or position of the laser incisions (C) or after surgical therapy, it releases the immobilization of the eye (3).

8. Ophthalmologic laser system (1) according to one of the preceding claims, **characterized by** a lock-in amplifier (13) for the detector (12), said lock-in amplifier (13) being coupled to the laser (4).

9. Computer program or control unit (14) for an ophthalmologic laser system (1) whose laser (4) is switchable between an illumination laser power and a therapy laser power and whose laser light is focusable three-dimensionally in a variable manner in a cornea (2), according to one of the preceding claims, configured to carry out the following steps:
- immobilization of an eye (3), containing the cornea (2), by means of an immobilization device (17),
- irradiation of a cornea (2), arranged in the examination area, by means of the laser (4) with an illumination laser power, and mapping of detection light by means of the detector (12), wherein the cornea (2) is scanned three-dimensionally by being irradiated at several points and by detection light being mapped,
- identification of a laser incision (C) in the cornea (2), and determination of the form and/or position of the laser incision (C) on the basis of the detection light at the scanning points (P), wherein scanning points are defined as support locations of the laser incision (C),
- determination of irradiation control data for refractive surgical follow-up treatment, wherein the determined form and/or position of the laser incisions (C) is taken into account by adapting a model of the laser incision (C) to the three-dimensional coordinates of the determined support locations in a compensating calculation,
- irradiation of the cornea (2) by means of the laser (4) with a surgical therapy laser power in accordance with the determined irradiation control data, and
- release of the immobilization of the eye (3).

10. Computer program or control unit (14) according to Claim 9, wherein two consecutive scanning points (P) differ from each other in all three spatial coordinates.

11. Computer program or control unit (14) according to Claim 9 or 10, configured to adjust a pulse frequency of the laser light in accordance with the speed of movement of a focal point of the laser beam relative to the cornea (2).

12. Computer program or control unit (14) according to one of Claims 9 to 11, wherein configured to carry out the step of irradiating and scanning the cornea along a scan curve several times, wherein the scan curve is used in each of the scanning passes in a differently offset and/or differently rotated position.

13. Computer program or control unit (14) according to one of Claims 9 to 12, configured to subtract a dark-field value from mapped detection light.

## Revendications

1. Système laser ophtalmologique (1), notamment à des fins d'un traitement de suivi chirurgical de la cornée, comprenant un laser (4) dont le rayonnement, en tant que lumière d'éclairage, peut être concentré dans une zone d'examen par le biais d'un trajet de faisceau d'éclairage (B) qui possède une unité de balayage (7) et une optique de concentration (8), comprenant un séparateur de faisceau (5) dans le trajet de faisceau d'éclairage (B), le rayonnement qui atteint le séparateur de faisceau (5) depuis la direction de la zone d'examen passant à travers un diaphragme d'ouverture (11) confocal pour arriver sur un détecteur (12) sous la forme de lumière de détection, et une unité de commande qui est conçue pour :
- irradier au moyen du laser (4) une cornée (2) disposée dans la zone d'examen avec une puissance laser d'éclairage et capture de la lumière de détection au moyen du détecteur (12), l'unité de commande (14) palpant la cornée (2) en trois dimensions en l'irradiant en plusieurs points et en capturant de la lumière de détection,
**caractérisé en ce que** l'unité de commande est conçue pour :
- identifier une coupe au laser (C) dans la cornée (2) et déterminer la forme et/ou la position de la coupe au laser (C) à l'aide de la lumière de détection.

2. Système laser ophtalmologique (1) selon la revendication 1, **caractérisé en ce que** le séparateur de faisceau (5) est un séparateur de faisceau à polarisation qui découple la lumière de détection de manière sélective sur le détecteur (12) de telle sorte qu'il présente une direction de polarisation différente de la lumière d'éclairage émise.

3. Système laser ophtalmologique (1) selon la revendication 1 ou 2, **caractérisé en ce qu'**un système optique de retard de phase (9) est disposé dans le trajet de faisceau d'éclairage (B) entre l'optique de concentration (8) et la zone d'examen de telle sorte que la lumière d'éclairage qui passe à travers reçoit une direction de polarisation correspondant à celle de la lumière de détection découplée.

4. Système laser ophtalmologique (1) selon la revendication 1, **caractérisé en ce qu'**un élément optique de polarisation qui, du point de vue d'une direction de polarisation, est solidaire en rotation ou rotatif, est disposé dans le trajet de rayon de détection (D) entre le séparateur de faisceau (5) et le détecteur (12).

5. Système laser ophtalmologique (1) selon l'une des revendications 1 à 4, **caractérisé en ce que** le rayonnement du laser (4), outre la puissance laser d'éclairage, peut être réglé à une puissance laser de thérapie chirurgicale.

6. Système laser ophtalmologique (1) selon la revendication 5, **caractérisé en ce que** les étapes suivantes peuvent être exécutées au moyen de l'unité de commande (14), après avoir déterminé la forme et/ou la structure de la cornée (2) :
- détermination de données de commande d'irradiation pour un traitement de suivi chirurgical réfractif, l'unité de commande (14) tenant compte de la forme et/ou de la position déterminées des coupes au laser (C), et
- irradiation de la cornée (2) à une puissance laser de thérapie chirurgicale conformément aux données de commande d'irradiation déterminées.

7. Système laser ophtalmologique (1) selon l'une des revendications précédentes, **caractérisé en ce que** l'unité de commande (14) immobilise un oeil (3) contenant la cornée (2) avant l'irradiation à la puissance laser d'éclairage au moyen d'un dispositif d'immobilisation (17) et relâche l'immobilisation de l'oeil (3) après la détermination de la forme et/ou de la position des coupes au laser (C) ou après une thérapie chirurgicale.

8. Système laser ophtalmologique (1) selon l'une des revendications précédentes, **caractérisé par** un amplificateur synchrone (13) couplé avec le laser (4) pour le détecteur (12).

9. Programme informatique ou unité de commande (14) pour un système laser ophtalmologique (1) dont le laser (4) peut être permuté entre une puissance laser d'éclairage et une puissance laser de thérapie et dont la lumière laser peut être concentrée dans une cornée (2) avec une variabilité en trois dimensions, selon l'une des revendications précédentes, conçu pour l'exécution des étapes suivantes :
- immobilisation d'un oeil (3) contenant la cornée (2) au moyen d'un dispositif d'immobilisation (17),
- irradiation d'une cornée (2) disposée dans la zone d'examen au moyen du laser (4) avec une puissance laser d'éclairage et capture de la lumière de détection au moyen du détecteur (12), la cornée (2) étant palpée en trois dimensions en ce qu'elle est irradiée en plusieurs points et la lumière de détection est capturée,
- identification d'une coupe au laser (C) dans la cornée (2) et détermination de la forme et/ou de la position de la coupe au laser (C) à l'aide de la lumière de détection aux points de palpage (P), les points de palpage étant définis comme des points de repère de la coupe au laser (C),
- détermination de données de commande d'irradiation pour un traitement de suivi chirurgical réfractif, la forme et/ou la position déterminées des coupes au laser (C) étant prises en compte en ce qu'un modèle de la coupe au laser (C) est adapté dans un calcul de compensation aux coordonnées tridimensionnelles des points de repère déterminés, et
- irradiation de la cornée (2) au moyen du laser (4) à une puissance laser de thérapie chirurgicale conformément aux données de commande d'irradiation déterminées et
- libération de l'immobilisation de l'oeil (3).

10. Programme informatique ou unité de commande (14) selon la revendication 9, deux points de palpage (P) successifs se différenciant dans les trois coordonnées spatiales.

11. Programme informatique ou unité de commande (14) selon la revendication 9 ou 10, conçu pour régler une fréquence d'impulsion de la lumière laser en fonction de la vitesse de déplacement d'un point focal du rayon laser par rapport à la cornée (2).

12. Programme informatique ou unité de commande (14) selon l'une des revendications 9 à 11, conçus pour exécuter plusieurs fois l'étape d'irradiation et de palpage de la cornée le long d'une courbe de palpage, la courbe de palpage étant respectivement utilisée avec une position décalée différente et/ou une position tournée différente dans les passages de palpage.

13. Programme informatique ou unité de commande (14) selon l'une des revendications 9 à 12, conçus pour retirer une valeur de fond noir de la lumière de détection capturée.
